# EUROPÄISCHE PATENTANMELDUNG

(11) **EP 1 023 911 A2**
(43) Veröffentlichungstag der Anmeldung: **02.08.2000**
(21) Anmeldenummer: 00101291.3
(22) Anmeldetag: 27.01.2000
(51) Int. Cl.: A61M 15/06, B05B 7/00

(54) **Vernebler, insbesondere zu Inhalationszwecken**

(30) Priorität: 28.01.1999 DE 19903374
(71) Anmelder: PARI GmbH Spezialisten für effektive Inhalation, 82319 Starnberg (DE)
(72) Erfinder: Ruf, Hartwig, 59909 Bestwig/Ostwig (DE); Waldner, Robert, 86971 Peiting (DE); Ott, Oskar, 82327 Tutzing/Kampenberg (DE); Sommer, Eric, 82205 Gilching (DE)
(74) Vertreter: HOFFMANN - EITLE

(57) **Zusammenfassung**

Bei dem erfindungsgemäßen Vernebler ist die Gestaltung der Verneblerdüse, die einen Druckgaskanal 4 und einen Flüssigkeitskanal 6 umfaßt, abgestimmt mit der Gestaltung eines Impaktionsrohres 3 und eines in Verlängerung dazu ausgebildeten Austrittsstutzens 10. Gegenläufige Effekte bei der Erzeugung des Flüssigkeitsnebels und bei dessen Herausführen durch das Impaktionsrohr und den Austrittsstutzen führen zu einer Ausfilterung zu großer Tröpfchen, so da der Patient einen Flüssigkeitsnebel, bestehend aus Tröpfchen in einem ausgewählten Größenbereich einatmet.

## Beschreibung

Die Erfindung betrifft einen Vernebler für die Vernebelung von flüssigen Stoffen, wie Medikamenten, Heilwässern oder dgl., insbesondere zu Inhalationszwecken.

Vernebler oder Zerstäuber für Inhalationszwecke sind in verschiedenster Ausgestaltung bekannt. Bei einigen Inhalationsverneblern wird besonderes Augenmerk darauf gerichtet, daß besonders kleine Tröpfchen im lungengängigen Bereich, d.h. in der Größenordnung von 0,5 bis 5 µm erzeugt werden. Diese besonders kleinen Tröpfchen eignen sich für die Deposition eines Medikaments in der Tiefe der Atemwege. Bei anderen Inhalationsverneblern steht die Erzeugung eines Aerosols im Vordergrund, dessen Tröpfchen eine Größe besitzen, die zu einer Deposition insbesondere im Bereich der oberen Atemwege des Patienten führt. Diese Art der Deposition wird erreicht bei Tröpfchen im Bereich von 5 bis 50µm. Die unterschiedlichen Anforderungen führen dazu, daß trotz ähnlichem Aufbaus Konstruktionsdetails von der einen Verneblerart auf die andere Verneblerart nicht ohne weiteres übertragen werden können.

Ein Vernebler der zuletzt angesprochenen Art ist bekannt aus DE 30 43 537 A. Bei diesem Vernebler ist in einem Verneblungsraum eine Düse angeordnet, die aus einem Druckluftkanal und einem Flüssigkeitskanal zusammengesetzt ist. Der Druckluftkanal verläuft an seinem Ende parallel zur Längsachse eines Austrittsstutzens und öffnet sich zu einer senkrecht zu dieser Achse verlaufenden Mündungsebene. Parallel zur Längsachse des Druckluftkanals verläuft die Mündungsebene des Flüssigkeitskanals, der an seinem Ende senkrecht zu dieser Ebene ausgerichtet ist. Durch die aus der Luftaustrittsöffnung austretendene Druckluft wird über den Flüssigkeitskanal die zu zerstäubende Flüssigkeit angesaugt (Bernoulli-Prinzip) und im Druckluftstrom zerstäubt. Der dadurch in der Druckluft entstehende Flüssigkeitsnebel wird über den Austrittsstutzen aus dem Verneblungsraum in Richtung der Längsachse des Austrittsstutzens herausgeführt, die vertikal verläuft, wenn der bekannte Vernebler in Gebrauchslage aufgestellt ist.

Eine ebenfalls nach dem Bernoulli-Prinzip arbeitende Düse mit ähnlichem Aufbau ist aus WO 97/11783 bekannt. Bei dieser Düse sind die Mündungsebenen des Druckluftkanals und des Flüssigkeitskanals parallel zueinander ausgerichtet, so daß die Mündungsebene des Flüssigkeitskanals in Richtung der austretenden Druckluft ein wenig stromabwärts und somit ausgehend von Gebrauchslage der Düse oberhalb der Mündungsebene des Druckluftkanals angeordnet ist. Der schräg zum Druckluftkanal verlaufende Flüssigkeitskanal gestattet eine Überlappung zwischen dem Flüssigkeitskanal und dem Druckluftkanal im Bereich der Mündungsebenen, wodurch eine besonders wirksame Ablösung der Flüssigkeit an der scharfen Kante des Flüssigkeitskanals erreicht werden soll.

Bei den hier diskutierten und zuvor anhand von Beispielen beschriebenen Verneblern steht die Erzeugung von Tröpfchen im Vordergrund, die sich im oberen Bereich der Atemwege des Patienten ablagern und die deshalb eine dafür geeignete Größe aufweisen müssen. Um dem Patienten eine ausreichende Menge atenibarer Tröpfchen zur Verfügung zu steilen, muß einerseits die Deposition der Tröpfchen in den zum Patienten führenden Aerosolzuleitungen besonders gering gehalten werden. Andererseits sollten aber zu große Tröpfchen (>50µm), die bei der Deposition in den oberen Atemwegen als unangenehm empfunden werden, sich in den Zuleitungen niederschlagen, so daß sie dem Patienten nicht dargeboten werden.

Vor diesem Hintergrund besteht das technische Problem der Erfindung darin, einen Vernebler der eingangs geschilderten Art so auszugestalten, daß dem Patienten ein atembarer Flüssigkeitsnebel mit der gewünschten Tröpfchengröße (5 bis 50µm) in der gewünschten Menge und unter Vermeidung zu großer Tröpfchen dargeboten wird.

Gelöst wird dieses Problem durch einen Vernebler mit einem Verneblungsraum, mit einem rohrförmigen Impaktionsstutzen, der sich in den Verneblungeraum hinein erstreckt, mit einer Verneblerdüse mit einem Druckluftkanal, dessen Längsachse zumindest in einem Endabschnitt des Druckluftkanals im wesentlichen parallel zur Längsachse des Impaktionsstutzens verläuft und der in einer im wesentlichen senkrecht zur Längsachse des Impaktionsstutzens verlaufenden ersten Mündungsebene eine Luftaustrittsöffnung bildet, und einem Flüssigkeitskanal, dessen Längsachse zumindest in einem Endabschnitt des Flüssigkeitskanals geneigt zur Längsachse des Druckluftkanals verläuft und der in einer im wesentlichen parallel zur Längsachse der Druckluftzuleitung verlaufenden zweiten Mündungsebene eine Flüssigkeitsaustrittsöffnung bildet, und mit einem rohrförmigen Austrittsstutzen, der in Verlängerung des Impaktionsstutzens ausgebildet ist, dessen Längsachse zur Längsachse des Impaktionsstutzens geneigt ist und der auf derselben Seite der zweiten Mündungsebene wie der Endabschnitt des Flüssigkeitskanals angeordnet ist.

In einer vorteilhaften Ausgestaltung sind die Längsachsen des Flüssigkeitskanals und des Austrittsstutzens im wesentlichen in derselben Ebene angeordnet. Diese besonders vorteilhafte Gestaltung zeichnet sich aus durch einen symmetrischen Aufbau, der die Erzeugung des gewünschten Flüssigkeitsnebels unterstützt.

In einer weiteren vorteilhaften Ausgestaltung ist die zweite Mündungsebene bezüglich des Druckluftkanals derart angeordnet, daß sie innerhalb des kegelförmigen Profils der aus dem Druckluftkanal austretenden Druckluft liegt.

In einer weiteren Ausgestaltung ist die zweite Mündungsebene zu der Längsachse des Druckluftkanals in einem Abstand angeordnet, der maximal dem Durchmesser der Druckluftaustrittsöffnung in der ersten Mündungsebene entspricht. Ein größerer Abstand kann zu einer unzureichenden Ausbildung des Bernoulli-Effekts führen, so daß nicht ausreichend große Tröpfchenmengen erzeugt werden.

In einer bevorzugten Ausgestaltung ist die zweite Mündungsebene zu der Längsachse des Druckluftkanals in einem Abstand angeordnet, der maximal dem halben Durchmesser der Druckluftaustrittsöffnung in der ersten Mündungsebene entspricht.

Um eine Druckluftquelle einfach an den erfindungsgemäßen Vernebler anschließen zu können, ist vorteilhaft der Druckluftkanal aus dem Verneblungsraum herausgeführt.

An dem in Gebrauchslage unteren Ende ist bei dem erfindungsgemäßen Vernebler vorzugsweise ein Flüssigkeitsreservoir vorgesehen. Für das Ansaugen von Flüssigkeit erstreckt sich der Flüssigkeitskanal in das Flüssigkeitsreservoir im Inneren des Verneblers.

Bei dem erfindungsgemäß ausgestalteten Vernebler ist besonders vorteilhaft, daß durch die Anordnung der Verneblerdüse in Bezug auf das Impaktionsrohr eine frühzeitige Abscheidung zu großer Tröpfchen erreicht wird, wenn der Patient den erzeugten Flüssigkeitsnebel über den Austrittsstutzen bzw. das daran angebrachte Mundstück einatmet. Denn die bevorzugte Ausbreitungsrichtung des von der Düse erzeugten Sprühnebels führt dazu, daß zu große Tröpfchen ihre ursprüngliche bevorzugte Ausbreitungsrichtung in einem Umfang beibehalten, daß es zu einem Niederschlagen an der Innenwand des Impaktionsstutzens kommt, während Tröpfchen in dem gewünschten Größenbereich während des Einatmens durch den entstehenden Luftstrom umgelenkt werden und durch den Austrittsstutzen hindurch zum Patienten gelangen. Außerhalb der Atmungsvorgänge wird ein sehr großer Anteil der Tröpfchen die ursprüngliche Ausbreitungsrichtung beibehalten. Zu einem frühen Zeitpunkt treffen Tröpfchen an der Innenwand des Impaktionsstutzens auf und werden in das Flüssigkeitsreservoir zurückgeführt. Ferner erfolgt die Einwirkung des im Ansaugstutzen erzeugten Luftstroms auf die Tröpfchen des erzeugten Flüssigkeitsnebels in entgegengesetzte Richtung zu der durch die Düse wirksam vorgegebenen bevorzugten Ausbreitungsrichtung der Tröpfchen. Flüssigkeitströpfchen in dem gewünschten Größenbereich werden dadurch in ausreichendem Maß so beeinflußt, daß sie während des Einatmens durch den Impaktionsstutzen und den Austrittsstutzen bis zum Patienten bewegt werden, wohingegen zu große Tröpfchen trotz der Einwirkung des Luftstroms ihre ursprüngliche Ausbreitungsrichtung beibehalten, die zu einem Kontakt mit der Innenwand des Impaktionsstutzens und zu einem Niederschlagen und Zurückführen in ein Flüssigkeitsreservoir führt. Uberraschenderweise wird die Größenverteilung des Primäraerosols positiv beeinflußt, wenn die erfindungsgemäße Anordnung der Mündungsebenen gewählt wird und Druckluftkanal und Flüssigkeitskanal schräg zueinander verlauten.

Bei dem erfindungsgemäßen Vernebler ist somit die Gestaltung der Verneblerdüse, die einen Druckgaskanal und einen Flüssigkeitskanal umfaßt, abgestimmt mit der Gestaltung des Impaktionsrohres und des in Verlängerung dazu ausgebildeten Austrittsstutzens. Gegenläufige Effekte bei der Erzeugung des Flüssigkeitsnebels und bei dessen Herausführen durch das Impaktionsrohr und den Austrittsstutzen führen zu einer Ausfilterung zu großer Tröpfchen, so daß der Patient einen Flüssigkeitsnebel, bestehend aus Tröpfchen in einem ausgewählten Größenbereich einatmet.

Im folgenden wird die Erfindung anhand eines Ausführungsbeispiels und unter Bezugnahme auf die beigefügten Zeichnungen genauer beschrieben, in denen zeigt:
- Fig. 1: einen erfindungsgemäßen Vernebler in Schnittdarstellung und
- Fig. 2: die Darstellung des entstehenden Flüssigkeitsnebels anhand einer schematisch wiedergegebenen Düse.

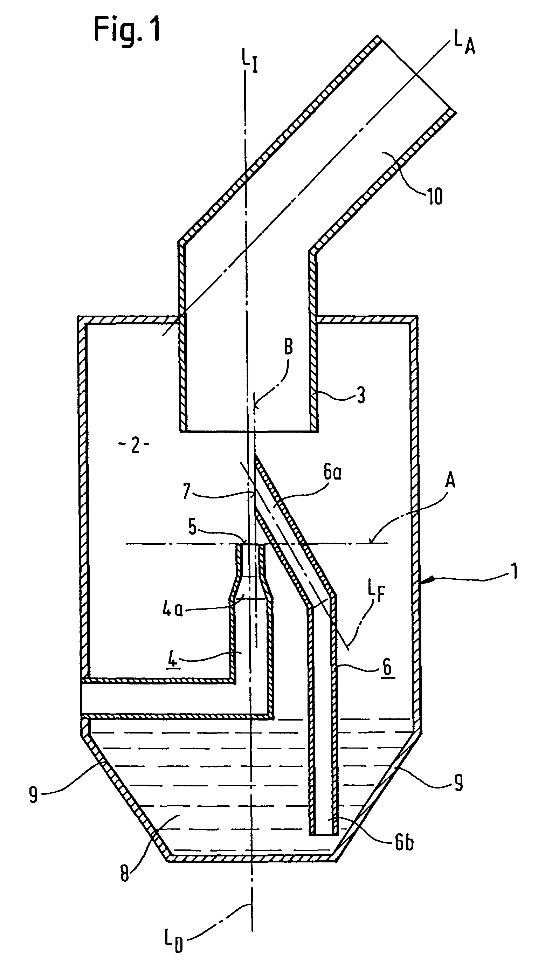

In Fig. 1 ist ein erfindungsgemäßer Vernebler 1 dargestellt, der einen Verneblungsraum 2 aufweist, in dem der Flüssigkeitsnebel erzeugt wird. In den Verneblungsraum 2 erstreckt sich ein rohrförmiger Impaktionsstutzen 3, der bei dem hier erläuterten Ausführungsbeispiel kreiszylindrisch ist. Koaxial zur Längsachse L_{I} des rohrtörmigen Impaktionsstutzens 3 ist die Längsachse L_{D} eines Druckluftkanals 4 angeordnet, der, wie beispielsweise in Fig. 1 gezeigt, aus dem Verneblungsraum hinausgeführt ist, um an eine Druckluftquelle (nicht dargestellt) angeschlossen werden zu können. Der Endabschnitt 4a des Druckluftkanals 4, dessen Längsachse L_{D} zumindest parallel zur Längsachse L_{I} des Impaktionsstutzen 3 angeordnet ist, bildet in der Mündungsebene A eine Druckluftaustrittsöffnung 5, aus der die durch den Druckluftkanal 4 zugeführte Druckluft ausströmt. Die ausströmende Druckluft weist typischerweise ein kegelförmiges Profil aus.

Wie aus Fig. 1 hervorgeht, ist neben dem Druckluftkanal 4 ein Flüssigkanal 6 vorgesehen, der zumindest einen Endabschnitt 6a besitzt, dessen Längsachse L_{F} schräg zur Längsachse L_{D} des Druckluftkanals 4 verläuft. Die Mündungsebene B des Flüssigkeitskanals 6 verläuft senkrecht zur Mündungsebene A des Druckluftkanals 4 und somit parallel zur Längsachse L_{D} des Druckluftkanals 4 bzw. zur Längsachse L_{I} des Impaktionsrohres 3. Die Mündungsebene B des Flüssigkeitskanals 6 ist von der Längsachse L_{D} des Druckluftkanals 4 in einem Abstand angeordnet, der beispielsweise dem Durchmesser des Druckluftkanals in der Mündungsebene A entspricht. Vorzugsweise ist der Abstand kleiner oder gleich dem halben Durchmesser des Druckluftkanals. Wesentlich ist hierbei, daß die Mündungsebene B innerhalb des kegelförmigen Profils der aus dem Druckluftkanal 4 ausströmenden Druckluft liegt. Der Flüssigkeitskanal 6 bildet in der Mündungsebene B eine Flüssigkeitsaustrittsöffnung 7, die bei einer vorzugsweise kreiszylindrischen Ausgestaltung des Flüssigkeitskanals 6 einen elliptischen Querschnitt in der Mündungsebene B besitzt.

Die aus dem Druckluftkanal 4 austretende Druckluft strömt an der Flüssigkeitsaustrittsöffnung 7 des Flüssigkeitskanals 6 vorbei und bewirkt aufgrund des Bernoulli-Prinzips ein Ansaugen der Flüssigkeit durch den Flüssigkeitskanal. Die Versorgung mit Flüssigkeit ist sichergestellt, indem, wie beispielsweise in Fig. 1 gezeigt, der Flüssigkeitskanal 6 in ein Flüssigkeitsreservoir 8 ragt, das bezogen auf die Gebrauchslage des Verneblers in einem unteren Bereich der Verneblers derart ausgebildet ist, daß die Gestaltung der Wände 9 die Ansammlung der Flüssigkeit im Bereich des Ansaugen 6b des Flüssigkeitskanals unterstützen.

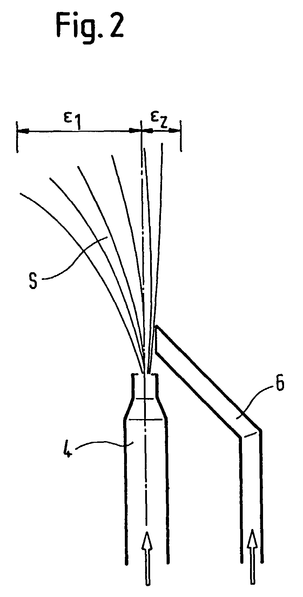

Die über den Flüssigkeitskanal angesaugte Flüssigkeit wird von der an der Flüssigkeitsaustrittsöffnung vorbeistreifenden Druckluft mitgerissen und zerstäubt. Aufgrund der Anordnung des Flüssigkeitskanals 6 im Bereich des austretenden Luftstroms erfährt dieser eine Störung, die eine Richtungsänderung und Aufweitung des Strahls bewirkt. Daraus resultiert die charakteristische Form des Sprühkegels S, die in Fig. 2 bei einer vereinfacht dargestellten Düse wiedergegeben ist. Die hier erkennbare bevorzugte Ausbreitungsrichtung im Bereich ε₁ des Sprühnebels führt zu einer frühzeitigen Impaktion größer Tröpfchen an dem in den Verneblungsraum ragenden Impaktionsrohr 3, wie anhand von Fig. 1 in Verbindung mit Fig. 2 leicht nachvollzogen werden kann. Die an der Innenwand des Impaktionsstutzens aufprallenden Tröpfchen werden sich regelmäßig an der Innenwand niederschlagen und von dort aufgrund der in Gebrauchslage wirkenden Schwerkraft in das Flüssigkeitsreservoir 8 am unteren Ende des Verneblers zurückgeführt.

Das frühzeitige Niederschlagen und Zurückführen großer Tröpfchen führt einerseits dazu, daß diese Tröpfchen nicht aus dem Vernebler hinausgetragen werden, wenn der Patient die Erzeugung des Flüssigkeitsnebels ausgelöst hat, den Flüssigkeitsnebel aber nicht einatmet. Das rasche Niederschlagen und Zurückführen bewirkt zusätzlich, daß bei der in der Regel temperierten Medikamentenflüssigkeit keine zu große Abkühlung stattfindet, da die zurückgeführten Flüssigkeitsmengen nur über einen geringen Zeitraum abgekühlt wurden.

Bei dem erfindungsgemäßen Vernebler ist in Verlängerung des Impaktionsstutzens 3 ein Ansaugstutzen 10 vorgesehen, dessen Längsachse L_{A} geneigt zur Längsachse L_{I} des Impaktionsrohres 3 verläuft. Dabei ist der Austrittsstutzen 10 so angeordnet, daß er auf derselben Seite der Mündungsebene B wie der Flüssigkeitskanal 6 liegt. Dadurch wird erreicht, daß beim Einatmen des Flüssigkeitsnebels eine Strömung in der Aerosolzuleitung zum Patienten, d.h. im Impaktionsstutzen 3 und Austrittsstutzen 10, entsteht, die die bevorzugte Ausbreitung der Flüssigkeitströpfchen einschränkt und die Richtung der Tröpfchenbewegung beeinflußt. Das Ausmaß der Beeinflussung hängt von der Größe der Tröpfchen ab, da die Masse der Tröpfchen ausschlaggebend für den Umfang der Bewegungsänderung der Tröpfchen aufgrund des Luftstroms in der Aerosolzuleitung ist. Durch die Gestaltung des Impaktionsstutzens 3 und des Austrittsstutzens 10, insbesondere durch die Neigung des Anschlußstutzens 10 bezogen auf den Impaktionsstutzen 3, können Tröpfchen eines gewünschten Größenbereichs gezielt beeinflußt werden, um diese Tröpfchen bevorzugt so abzulenken, daß sie sich nicht mehr an der Innenwand des Impaktionsrohres 3 niederschlagen, sondern durch den Luftstrom beim Einatmen zum Patienten transportiert werden.

## Patentansprüche

1. Vernebler mit
- einem Verneblungsraum (2),
- einem rohrförmigen Impaktionsstutzen (3), der sich in den Verneblungsraum hinein erstreckt,
- einer Verneblerdüse mit
-- einem Druckluftkanal (4), dessen Längsachse (L_{D}) zumindest in einem Endabschnitt (4a) des Druckluftkanals im wesentlichen parallel zur Längsachse (L_{I}) des Impaktionsstutzens (3) verläuft und der in einer im wesentlichen senkrecht zur Längsachse (L_{I}) des Impaktionsstutzens (3) verlaufenden ersten Mündungsebene (A) eine Luftaustrittsöffnung (5) bildet,
-- einem Flüssigkeitskanal (6), dessen Längsachse (L_{F}) zumindest in einem Endabschnitt (6a) des Flüssigkeitskanals (6) geneigt zur Längsachse (L_{D}) des Druckluftkanals (4) verläuft und der in einer im wesentlichen parallel zur Längsachse (L_{D}) des Druckluftkanals (6) verlaufenden zweiten Mündungsebene (B) eine Flüssigkeitsaustrittsöffnung (7) bildet, und
- einen rohrförmigen Austrittsstutzen (10), der als Verlängerung des Impaktionsstutzens (3) ausgebildet ist, dessen Längsachse (L_{A}) zur Längsachse (L_{I}) des Impaktionsstutzens (3) geneigt ist und der auf derselben Seite der zweiten Mündungsebene (B) wie der Endabschnitt (6a) des Flüssigkeitskanals (6) angeordnet ist.

2. Vernebler nach Anspruch 1,
dadurch **gekennzeichnet,** daß
die Längsachsen (L_{F}, L_{A}) des Flüssigkeitskanals (6) und des Austrittsstutzens (10) im wesentlichen in derselben Ebene angeordnet sind.

3. Vernebler nach Anspruch 1 oder 2,
dadurch **gekennzeichnet,** daß
die zweite Mündungsebene (B) bezogen auf den Druckluftkanal (4) derart angeordnet ist, daß sie innerhalb des kegelförmigen Profils der aus dem Druckluftkanal(4) ausströmenden Druckluft liegt.

4. Vernebler nach Anspruch 3,
dadurch **gekennzeichnet,** daß
die zweite Mündungsebene (B) zu der Längsachse (L_{D}) des Druckluftkanals (4) in einem Abstand angeordnet ist, der maximal dem Durchmesser der Druckluftaustrittsöffnung (5) in der ersten Mündungsebene (A) entspricht.

5. Vernebler nach Anspruch 4,
dadurch **gekennzeichnet,** daß
die zweite Mündungsebene (B) zu der Längsachse (L_{D}) des Druckluftkanals (4) in einem Abstand angeordnet ist, der maximal dem halben Durchmesser der Druckluftaustrittsöffnung (5) in der ersten Mündungsebene (A) entspricht.

6. Vernebler nach einem der Ansprüche 1 bis 5,
dadurch **gekennzeichnet,** daß
der Druckluftkanal (4) für den Anschluß einer Druckluftquelle aus dem Verneblungsraum herausgeführt ist.

7. Vernebler nach einem der Ansprüche 1 bis 6,
dadurch **gekennzeichnet,** daß
sich der Flüssigkeitskanal (6) in ein Flüssigkeitsreservoir (8) im Inneren des Verneblers erstreckt.
